# EUROPÄISCHE PATENTSCHRIFT

(11) **EP 3 005 587 B1**
(45) Veröffentlichungstag und Bekanntmachung des Hinweises auf die Patenterteilung: **10.10.2018**
(21) Anmeldenummer: 14730439.8
(22) Anmeldetag: 20.05.2014
(51) Int. Cl.: H04B 10/114, G06F 19/00, G08C 23/04

(54) **VERFAHREN UND EINRICHTUNG ZUM FERNBETÄTIGTEN STEUERN VON MEDIZINISCHEN GERÄTEN MITTELS EINER FERNBEDIENUNGSVORRICHTUNG**
METHOD AND ARRANGEMENT FOR REMOTE CONTROLLING MEDICAL APPARATUS BY MEANS OF A REMOTE CONTROL DEVICE
PROCÉDÉ ET SYSTÈME DE COMMANDE À DISTANCE D'APPAREILS MÉDICAUX AU MOYEN D'UNE TÉLÉCOMMANDE

(30) Priorität: 06.06.2013 DE 102013105822
(43) Veröffentlichungstag der Anmeldung: 13.04.2016
(73) Patentinhaber: Maquet GmbH, 76437 Rastatt (DE)
(72) Erfinder: RUCH, Jürgen, 77652 Offenburg (DE); HIRTH, Andreas, 76437 Rastatt (DE)
(74) Vertreter: Zacco GmbH
(86) Internationale Anmeldenummer: PCT/EP2014/060330
(87) Internationale Veröffentlichungsnummer: WO 2014/195126

(56) Entgegenhaltungen:
- US-A1- 2008 312 512
- US-A1- 2009 063 187
- US-A1- 2012 185 267

## Beschreibung

Die Erfindung betrifft ein Verfahren zum fernbetätigten Steuern von medizinischen Geräten mittels einer Fernbedienungsvorrichtung, bei dem zwischen einer in der Fernbedienungsvorrichtung enthaltenen ersten Infrarot-Sende-Empfangs-Einheit und einer in dem jeweiligen medizinischen Gerät enthaltenen zweiten Infrarot-Sende-Empfangs-Einheit ein gepaarter Betriebszustand hergestellt wird, in dem eine von der ersten Infrarot-Sende-Empfangs-Einheit an die zweite Infrarot-Sende-Empfangs-Einheit gesendete Fernbedienungsadresse gleich einer für die zweite Infrarot-Sende-Empfangs-Einheit voreingestellten Geräteadresse ist; das Vorliegen des gepaarten Betriebszustands geprüft wird; und bei Vorliegen des gepaarten Betriebszustands das medizinische Gerät in Abhängigkeit eines Steuerbefehls gesteuert wird, der bei Betätigen der Fernbedienungsvorrichtung von der ersten Infrarot-Sende-Empfangs-Einheit an die zweite Infrarot-Sende-Empfangs-Einheit gesendet wird.

In der Medizintechnik kommen häufig mit Infrarotstrahlung arbeitende Fernbedienungsvorrichtungen zum Einsatz, um medizinische Geräte, z.B. Operationstische, fernbetätigt zu steuern. Hierzu verfügen die Fernbedienungsvorrichtung und das anzusteuernde medizinische Gerät über Infrarot-Sende-Empfangs-Einheiten, die über Infrarotsignale bei Bestehen einer Sichtverbindung miteinander kommunizieren. Um mit einer einzigen Fernbedienungsvorrichtung aus einer Vielzahl von medizinischen Geräten wahlweise nur eines dieser Geräte fernbetätigt anzusprechen, wird zwischen der Infrarot-Sende-Empfangs-Einheit der Fernbedienungsvorrichtung und der Infrarot-Sende-Empfangs-Einheit des anzusteuernden medizinischen Gerätes ein gepaarter Betriebszustand hergestellt, in dem eine von der Fernbedienungsvorrichtung an das medizinische Gerät gesendete Infrarot-Fernbedienungsadresse gleich einer Infrarot-Geräteadresse ist, die für das jeweilige medizinische Gerät individuell voreingestellt ist. Beispielsweise wird dieser gepaarte Betriebszustand hergestellt, indem über einen an der Fernbedienungsvorrichtung vorgesehenen Drehcodierschalter die Fernbedienungsadresse mit der für das anzusteuernde medizinische Gerät voreingestellten Geräteadresse gleichgesetzt wird. Alternativ kann die Paarung zwischen der Fernbedienungsvorrichtung und dem medizinischen Gerät auch über ein computergestütztes Menü erfolgen.

Bevor sich das medizinische Gerät über die Fernbedienungsvorrichtung fernbetätigt steuern lässt, wird geprüft, ob die Fernbedienungsvorrichtung und das medizinische Gerät in vorstehend beschriebenem Sinne miteinander gepaart sind. Ist dies der Fall, so lässt sich das medizinische Gerät über Steuerbefehle fernbetätigt steuern, welche die Infrarot-Sende-Empfangs-Einheit der Fernbedienungsvorrichtung an die Infrarot-Sende-Empfangs-Einheit des medizinischen Gerätes sendet, wenn die Fernbedienungsvorrichtung entsprechend betätigt wird.

Üblicherweise bleibt eine einmal hergestellte Paarung so lange bestehen, bis die Fernbedienungsvorrichtung mit einem anderen Gerät gepaart wird, d.h. die Fernbedienungsadresse manuell auf die Geräteadresse dieses anderen Gerätes umgestellt wird. Das Umstellen der Fernbedienungsvorrichtung ist insbesondere dann unkomfortabel, wenn diese Umstellung recht häufig vorgenommen werden muss. Dies ist beispielsweise der Fall, wenn mit einer einzigen Fernbedienungsvorrichtung, z.B. einem Fußschalter oder einem Handgerät, wahlweise eine Vielzahl von Operationstischen für den prä- oder postoperativen Lagerflächentransfer angesteuert werden soll. Ein Verfahren zum fernbetätigten Steuern von medizinischen Geräten nach dem Stand der Technik ist aus der US-A-2008/0312512 bekannt.

Aufgabe der Erfindung ist es, ein Verfahren zum fernbetätigten Steuern von medizinischen Geräten eingangs erläuterter Art so weiterzubilden, dass der gepaarte Betriebszustand zwischen der Fernbedienungsvorrichtung und dem gerade anzusteuernden medizinischen Gerät einfach und sicher herstellt werden kann.

Die Erfindung löst diese Aufgabe dadurch, dass der gepaarte Betriebszustand hergestellt wird, indem bei Inbetriebnahme der Fernbedienungsvorrichtung von der ersten Infrarot-Sende-Empfangs-Einheit ein Anforderungsbefehl an die zweite Infrarot-Sende-Empfangs-Einheit gesendet wird, bei Empfang des Anforderungsbefehls von der zweiten Infrarot-Sende-Empfangs-Einheit die voreingestellte Geräteadresse an die erste Infrarot-Sende-Empfangs-Einheit gesendet wird und die von der ersten Infrarot-Sende-Empfangs-Einheit empfangene Geräteadresse als Fernbedienungsadresse übernommen wird.

Das erfindungsgemäße Verfahren sieht also gleichsam eine automatische Paarung zwischen den Infrarot-Sende-Empfangs-Einheiten der Fernbedienungsvorrichtung und des anzusteuernden Gerätes vor. Sobald die Fernbedienungsvorrichtung in Betrieb genommen wird, fordert sie die Infrarot-Geräteadresse des von ihr angesprochenen Gerätes an. Diese Geräteadresse übernimmt dann die Fernbedienungsvorrichtung als Fernbedienungsadresse und verwendet sie für die weitere Kommunikation mit dem angesprochenen Gerät. Ein manuelles Einstellen der Fernbedienungsadresse auf die für das anzusteuernde Gerät fest vorgegebene Geräteadresse entfällt somit.

Die Erfindung sieht das Senden des Anforderungsbefehls an das angesprochene medizinische Gerät bei Inbetriebnahme der Fernbedienungsvorrichtung vor. Mit "Inbetriebnahme" ist dabei beispielsweise eine erstmalige Betätigung der Fernbedienungsvorrichtung gemeint, mit der das Gerät konkret angesteuert soll, um z.B. die Patientenlagerfläche des Operationstisches zu verstellen. Mit "Inbetriebnahme" kann jedoch auch beispielsweise das Einschalten der Fernbedienungsvorrichtung gemeint sein, also der Beginn der Speisung mit elektrischer Energie, sofern eine solche Einschaltfunktion bei der Fernbedienungsvorrichtung vorgesehen ist. Ebenso ist denkbar, unter "Inbetriebnahme" die erstmalige Betätigung der Fernbedienungsvorrichtung nach Ablauf einer vorbestimmten Zeitspanne zu verstehen. Diese Zeitspanne kann in Abhängigkeit der anzusteuernden Geräte und der durchzuführenden Prozesse beispielsweise so gewählt sein, dass sie typischerweise eine Zeit festlegt, nach deren Ablauf damit zu rechnen ist, dass ein anderes Gerät aus der Gerätegruppe mit der Fernbedienungsvorrichtung angesprochen werden soll.

Da mit der Fernbedienungsvorrichtung in Folge der erfindungsgemäßen automatischen Paarung jedes Gerät der im Einsatz befindlichen Gerätegruppe adressierbar ist, kann die Fernbedienungsvorrichtung z.B. bei Verlust oder Defekt einfach durch eine andere Fernbedienungsvorrichtung, die gerade für die dieselbe Gerätegruppe verwendet wird, ersetzt werden. So bleibt die Verfügbarkeit der Gerätegruppe problemlos erhalten.

Vorzugsweise wird das Vorliegen des gepaarten Betriebszustands geprüft, indem die Geräteadresse, nachdem sie von der ersten Infrarot-Sende-Empfangs-Einheit als Fernbedienungsadresse übernommen worden ist, von der zweiten Infrarot-Sende-Empfangs-Einheit zyklisch ausgesendet wird und von der Fernbedienungsvorrichtung geprüft wird, ob die erste Infrarot-Sende-Empfangs-Einheit die Geräteadresse zyklisch empfängt. Bei dieser Ausführungsform der Erfindung wird also die für das angesprochene Gerät voreingestellte Geräteadresse mit Inbetriebnahme der Fernbedienungsvorrichtung übernommen und dann zyklisch geprüft, ob die einmal hergestellte Paarung aufrechterhalten wird.

Das Vorliegen des gepaarten Betriebszustands kann zusätzlich oder alternativ zu der vorstehend genannten Ausführungsform dadurch geprüft werden, dass die Fernbedienungsvorrichtung erfasst, ob die erste Infrarot-Sende-Empfangs-Einheit keine andere als die übernommene Geräteadresse empfängt. Empfängt die Fernbedienungsvorrichtung beispielsweise gleichzeitig zwei verschiedene Geräteadressen, so kann in diesem Fall die Paarung der Fernbedienungsvorrichtung mit dem zunächst angesprochenen Gerät aufgehoben und damit die fernbetätigte Steuerung dieses Gerätes unmöglich gemacht werden. Dies ermöglicht eine besonders sichere Geräteansteuerung mittels der Fernbedienungsvorrichtung.

Das Vorliegen des gepaarten Betriebszustands kann zusätzlich oder alternativ auch geprüft werden, indem eine Bewegung der Fernbedienungsvorrichtung erfasst wird. Ist die Fernbedienungsvorrichtung beispielsweise als Fußschalter realisiert, so kann dieser Fußschalter einen Bewegungssensor aufweisen, der ein Anheben des Fußschalters vom Boden erfasst und ein entsprechendes Signal ausgibt, so dass die zunächst hergestellt Paarung zwischen Fernbedienungsvorrichtung und angesprochenem Gerät aufgehoben wird. Auch dies ist eine Sicherheitsmaßnahme, die eine zuverlässige und gefahrlose Gerätesteuerung ermöglicht.

Vorzugsweise wird die von der ersten Infrarot-Sende-Empfangs-Einheit übernommene Geräteadresse in der Fernbedienungsvorrichtung gespeichert. Bei einer nächsten Inbetriebnahme der Fernbedienungsvorrichtung kann so die bei der vorhergehenden Inbetriebnahme gespeicherte Geräteadresse von der ersten Infrarot-Sende-Empfangs-Einheit als Fernbedienungsadresse an die zweite Infrarot-Sende-Empfangs-Einheit gesendet werden. Ist die Fernbedienungsvorrichtung beispielsweise ein mit Bedientasten versehenes Handgerät, so wird bei dieser Weiterbildung die Fernbedienungsvorrichtung zunächst mit der zuletzt eingestellten Fernbedienungsadresse gestartet. Dies ermöglicht eine besonders kurze Reaktionszeit, sofern mit der Fernbedienungsvorrichtung immer noch dasselbe Gerät angesteuert werden soll.

Vorzugsweise wird für den Fall, dass die bei der nächsten Inbetriebnahme der Fernbedienungsvorrichtung an die zweite Infrarot-Sende-Empfangs-Einheit gesendete Fernbedienungsadresse verschieden von der für die zweite Infrarot-Sende-Empfangs-Einheit individuell voreingestellten Geräteadresse ist, von der Fernbedienungsvorrichtung eine Benutzeraufforderung ausgegeben, die Geräteadresse zu bestätigen. Im Falle der Bestätigung wird dann die Geräteadresse von der ersten Infrarot-Sende-Empfangs-Einheit als Fernbedienungsadresse übernommen. Wird also in dieser Ausführungsform festgestellt, dass das gerade angesprochene Gerät mit einer anderen als der bisher verwendeten Geräteadresse antwortet, so wird der Benutzer aufgefordert, die neu erkannte Geräteadresse erst zu bestätigen, sofern er das Gerät, dem diese neue Geräteadresse zugeordnet ist, bedienen will. Auch dies erhöht die Sicherheit der fernbetätigten Gerätesteuerung.

Vorzugsweise wird bei Vorliegen des gepaarten Betriebszustands bei jeder Betätigung der Fernbedienungsvorrichtung der Steuerbefehl zusammen mit der Fernbedienungsadresse von der ersten Infrarot-Sende-Empfangs-Einheit an die zweite Infrarot-Sende-Empfangs-Einheit gesendet. Bei jeder Betätigung kann so in dem angesprochenen Gerät geprüft werden, ob die empfangene Fernbedienungsadresse mit der voreingestellten Geräteadresse übereinstimmt. Somit wird bei jeder Betätigung der Fernbedienungsvorrichtung geprüft, ob die Bedienungsvorrichtung und das Gerät noch wirksam miteinander gepaart sind.

Nach einem weiteren Aspekt der Erfindung ist eine Einrichtung mit Mitteln vorgesehen, die angepasst sind, das erfindungsgemäße Verfahren durchzuführen.

Die Erfindung wird im Folgenden anhand der Figuren näher erläutert. Darin zeigen:
- Fig. 1: eine schematische Darstellung, die einen Fußschalter und einen Operationstisch in einem ersten Anwendungsbeispiel vor Inbetriebnahme des Fußschalters zeigt;
- Fig. 2: eine schematische Darstellung, die das erste Anwendungsbeispiel bei Inbetriebnahme des Fußschalters zeigt;
- Fig. 3: eine schematische Darstellung, die das erste Anwendungsbeispiel beim Aussenden eines Steuerbefehls an den Operationstisch zeigt;
- Fig. 4: eine schematische Darstellung, die das erste Anwendungsbeispiel beim Aussenden eines weiteren Steuerbefehls an den Operationstisch zeigt;
- Fig. 5: eine schematische Darstellung, die das erste Anwendungsbeispiel beim Aussenden eines weiteren Steuerbefehls in einem nicht gepaarten Betriebszustand zeigt;
- Fig. 6: eine schematische Darstellung, die ein zweites Anwendungsbeispiel vor Inbetriebnahme des Fußschalters zeigt;
- Fig. 7: eine schematische Darstellung, die das zweite Anwendungsbeispiel beim Empfang von Geräteadressen zweier Operationstische zeigt; und
- Fig. 8: eine schematische Darstellung, die das zweite Anwendungsbeispiel in einem nicht gepaarten Betriebszustand zeigt.

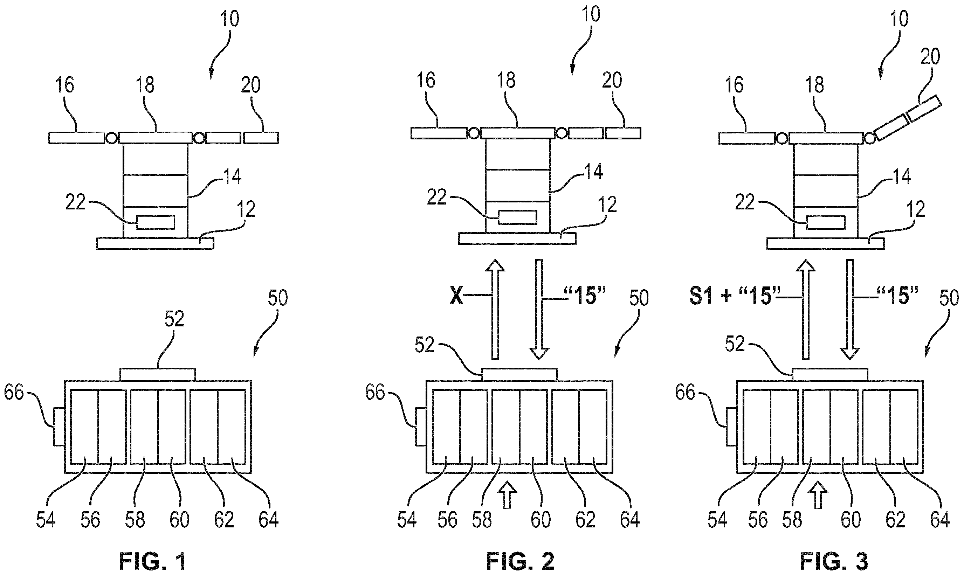

In den Figuren 1 bis 5 ist ein erstes Anwendungsbeispiel des erfindungsgemäßen Verfahrens veranschaulicht. In diesem ersten Anwendungsbeispiel soll ein Operationstisch 10 mittels einer als Fußschalter 50 ausgebildeten Fernbedienungsvorrichtung fernbetätigt gesteuert werden.

Der Operationstisch 10 weist eine Standfläche 12 und eine darauf aufsetzende Tischsäule 14 auf. Die Tischsäule 14 ist in vertikaler Richtung verstellbar. An dem oberen Ende der Tischsäule 14 ist eine aus mehreren gegeneinander verstellbaren Segmenten 16, 18 und 20 gebildete Patientenlagerfläche angebracht. Um die Tischsäule 14 und die Segmente 16, 18 und 20 der Patientenlagerfläche zu verstellen, verfügt der Operationstisch über eine in den Figuren nicht gezeigte Verstellmechanik. Zur fernbetätigten Steuerung dieser Verstellmechanik enthält die Tischsäule 14 eine Infrarot-Sende-Empfangs-Einheit 22.

Die Infrarot-Sende-Empfangs-Einheit 22 ist ausgebildet, über Infrarotsignale mit dem Fußschalter 50 zu kommunizieren. Hierzu verfügt der Fußschalter 50 über eine Infrarot-Sende-Empfangs-Einheit 52. Diese wird im Folgenden als erste Infrarot-Sende-Empfangs-Einheit bezeichnet, während die in dem Operationstisch 10 vorgesehene Einheit 22 als zweite Infrarot-Sende-Empfangs-Einheit bezeichnet wird.

Der Fußschalter 50 hat eine Reihe von Bedienelementen 54, 56, 58, 60, 62 und 64, die ein Benutzer mit dem Fuß betätigen kann, um den Operationstisch 10 fernbetätigt zu steuern. Im Einzelnen dient das Bedienelement 54 dazu, die erste Sende-Empfangs-Einheit 52 zu veranlassen, einen Steuerbefehl "HUB HOCH" an die zweite Infrarot-Sende-Empfangs-Einheit 22 zu senden. Der Steuerbefehl "HUB HOCH" soll ein Verfahren der Tischsäule 14 nach oben und damit ein Anheben der Patientenlagerfläche als Ganzes bewirken. Entsprechend ist das Bedienelement 56 einem Steuerbefehl "HUB TIEF" zugeordnet, durch den ein Absenken der Patientenlagerfläche veranlasst werden soll.

Das Bedienelement 58 ist einem Steuerbefehl "RÜCKEN HOCH" zugeordnet, der bewirken soll, dass das eine Rückenplatte bildende Segment 20 der Patientenlagerfläche nach oben geschwenkt wird. Entsprechend ist das Bedienelement 60 einem Steuerbefehl "RÜCKEN TIEF" zugeordnet. Dieser soll bewirken, dass das Segment 20 nach unten geschwenkt wird.

Das Bedienelement 62 ist einem Steuerbefehl "BEINE HOCH" zugeordnet. Durch diesen Steuerbefehl soll der Operationstisch veranlasst werden, das eine Beinplatte bildende Segment 16 der Patientenlagerfläche nach oben zu schwenken. Entsprechend ist dem Bedienelement 64 ein Steuerbefehl "BEINE TIEF" zugeordnet, der bewirken soll, dass das Segment 16 der Patientenfläche nach unten geschwenkt wird.

Der Fußschalter 50 weist ferner einen Bewegungssensor 66 auf, durch den ein Anheben des Fußschalters 50 vom Boden erfasst werden kann.

Figur 1 zeigt für das erste Anwendungsbeispiel den Zustand vor Inbetriebnahme des Fußschalters 50. In diesem Zustand ist der Fußschalter 50 in den OP-Raum gebracht und so auf dem Boden abgelegt worden, dass zwischen den beiden Infrarot-Sende-Empfangs-Einheiten 52 und 22 eine Sichtverbindung besteht. In diesem Zustand findet noch keine Kommunikation zwischen den beiden Infrarot-Sende-Empfangs-Einheiten 52 und 22 statt.

In Figur 2 betätigt der Benutzer mit seinem Fuß das Bedienelement 58, wodurch der Fußschalter 50 in Betrieb genommen wird. Durch Betätigen des Bedienelementes 58, dem der Steuerbefehl "RÜCKEN HOCH" zugeordnet ist, wird die erste Infrarot-Sende-Empfangs-Einheit 52 veranlasst, einen Anforderungsbefehl X an die zweite Infrarot-Sende-Empfangs-Einheit 22 zu senden. Durch den Anforderungsbefehl X wird die zweite Infrarot-Sende-Empfangs-Einheit 22 aufgefordert, eine auf den Operationstisch 10 bezogene Geräteadresse zurück an die erste Infrarot-Sende-Empfangs-Einheit 52 zu senden. Dabei ist die Geräteadresse speziell für den Operationstisch 10 voreingestellt. Andere (in den Figuren 1 bis 5 nicht gezeigte) Operationstische haben ebenfalls jeweils ihre eigene, von anderen Adressen unterscheidbare Geräteadresse.

Wie in Figur 2 gezeigt, sendet somit die zweite Infrarot-Sende-Empfangs-Einheit 22 die voreingestellte Geräteadresse, die hier rein beispielhaft mit "15" angegeben ist. Die von der ersten Infrarot-Sende-Empfangs-Einheit 52 empfangene Geräteadresse "15" wird in dem Fußschalter 50 als Fernbedienungsadresse übernommen. Somit sind die auf den Operationstisch bezogene Geräteadresse und die auf den Fußschalter 50 bezogene Fernbedienungsadresse gleich, wodurch ein gepaarter Betriebszustand zwischen den beiden Infrarot-Sende-Empfangs-Einheiten 52 und 22 hergestellt ist.

In dem in Figur 3 gezeigten Zustand bleibt das Bedienelement 58 weiterhin betätigt. Somit sendet die erste Infrarot-Sende-Empfangs-Einheit 52 den dem Bedienelement 58 zugeordneten Steuerbefehl "RÜCKEN HOCH", der in Figur 3 mit S1 bezeichnet ist, an die zweite Infrarot-Sende-Empfangs-Einheit 22 aus. Zusammen mit dem Steuerbefehl S1 sendet die erste Infrarot-Sende-Empfangs-Einheit 52 auch die von dem Operationstisch 10 empfangene und als Fernbedienungsadresse übernommene Infrarot-Adresse "15" aus. Der Operationstisch 10 erkennt den gepaarten Betriebszustand, d.h. die Übereinstimmung zwischen der voreingestellten Geräteadresse und der von dem Fußschalter 50 empfangenen Fernbedienungsadresse. Da somit der Operationstisch 10 und der Fußschalter 50 miteinander gepaart sind, führt die in dem Operationstisch 10 enthaltene Verstellmechanik den Steuerbefehl "RÜCKEN HOCH" (S1) aus, wodurch das Segment 20 der Patientenlagerfläche nach oben geschwenkt wird.

In dem in Figur 3 gezeigten Zustand sendet die zweite Infrarot-Sende-Empfangs-Einheit 22 zyklisch die Geräteadresse "15" an die erste Infrarot-Sende-Empfangs-Einheit 52. In dem Fußschalter 50 wird dementsprechend geprüft, ob die erste Infrarot-Sende-Empfangs-Einheit 52 die Geräteadresse "15" zyklisch empfängt.

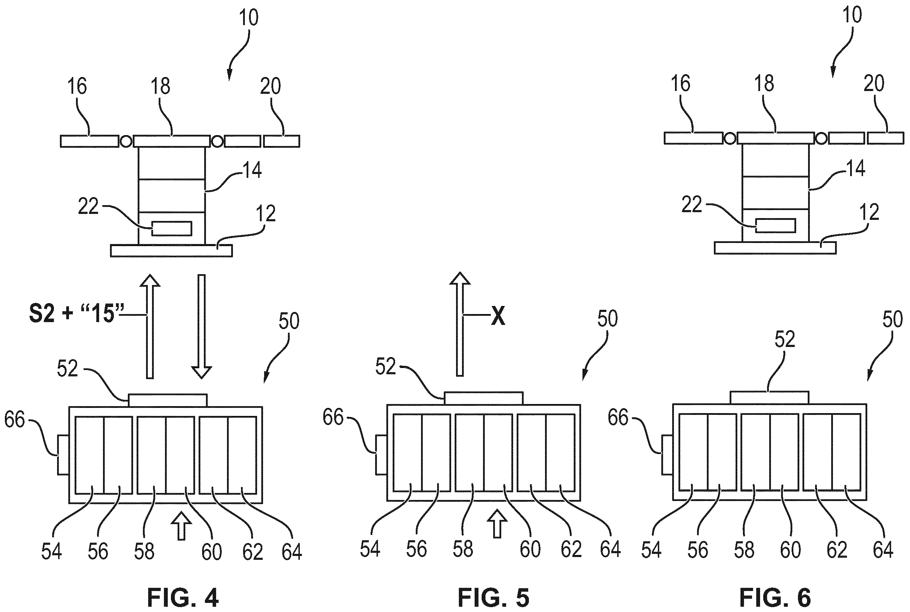

In Figur 4 betätigt der Benutzer ausgehend von dem in Figur 3 gezeigten Zustand nun das Bedienelement 60. Dementsprechend sendet die erste Infrarot-Sende-Empfangs-Einheit den dem Bedienelement 60 zugeordneten Steuerbefehl "RÜCKEN TIEF" zusammen mit der Fernbedienungsadresse "15" an die zweite Infrarot-Sende-Empfangs-Einheit 22 aus. Dieser Steuerbefehl ist in Figur 4 mit S2 bezeichnet. Dementsprechend wird die Verstellmechanik des Operationstisches 10 veranlasst, das Segment 20 der Patientenlagerfläche nach unten zu schwenken. Wie zudem in Figur 4 gezeigt, sendet die zweite Infrarot-Sende-Empfangs-Einheit 22 weiterhin zyklisch die Geräteadresse "15" an die erste Infrarot-Sende-Empfangs-Einheit 52.

In Figur 5 ist ein Zustand gezeigt, in dem der gepaarte Betriebszustand zwischen den beiden Infrarot-Sende-Empfangs-Einheiten 52 und 22 aufgehoben ist. Dies ist in dem in Figur 5 gezeigten Fall dadurch verursacht, dass keine Sichtverbindung mehr zwischen den beiden Infrarot-Sende-Empfangs-Einheiten 52 und 22 besteht und demnach die erste Infrarot-Sende-Empfangs-Einheit 52 die Geräteadresse nicht mehr zyklisch empfängt. Der gepaarte Betriebszustand kann aber beispielsweise auch dadurch beendet werden, dass der Fußschalter 50 von dem Boden angehoben und dies von dem Bewegungssensor 66 erfasst worden ist. In diesem Fall gibt der Bewegungssensor 66 ein entsprechendes Signal aus, durch das die Paarung der beiden Infrarot-Sende-Empfangs-Einheiten 52 und 22 aufgehoben wird. Da in dem in Figur 5 gezeigten Zustand keine Paarung mehr vorliegt, wird bei der nächsten Inbetriebnahme des Fußschalters 50, d.h. beispielsweise bei der nächsten Betätigung eines der Bedienelemente 54 bis 64, wiederum der Anforderungsbefehl X von der ersten Infrarot-Sende-Empfangs-Einheit 52 ausgesendet.

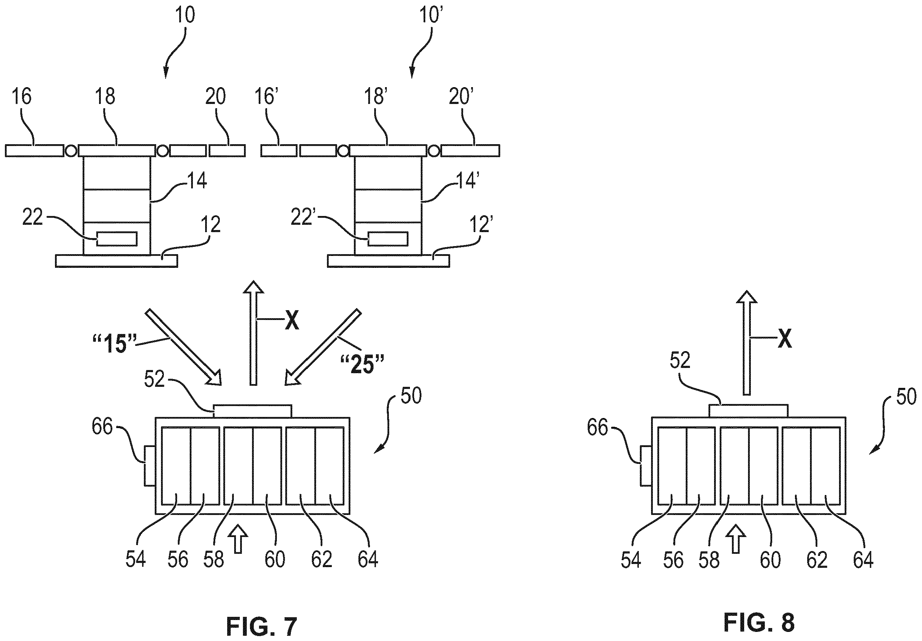

In den Figuren 6 bis 8 ist ein zweites Anwendungsbeispiel gezeigt. Dabei entspricht der Zustand nach Figur 6 dem in Figur 1 dargestellten Zustand. In Figur 7 betätigt der Benutzer wiederum mit seinem Fuß das Bedienelement 58, dem der Steuerbefehl "RÜCKEN HOCH" zugeordnet ist. Da durch diese Betätigung der Fußschalter 50 in Betrieb genommen wird, sendet die erste Infrarot-Sende-Empfangs-Einheit 52 zunächst den Anforderungsbefehl X aus, mit dem der angesprochene Operationstisch 10 zum Senden seiner Geräteadresse aufgefordert werden soll. Bei dem in Figur 7 gezeigten Beispiel wird dieser Anforderungsbefehl nicht nur von dem Operationstisch 10, sondern von einem weiteren Operationstisch 10' empfangen, der baugleich mit dem Operationstisch 10 ist, jedoch eine andere Geräteadresse, nämlich "25" aufweist. Dementsprechend empfängt die erste Infrarot-Sende-Empfangs-Einheit sowohl die Geräteadresse "15" als auch die Geräteadresse "25". Der Empfang zweier Geräteadressen durch den Fußschalter 50 führt dazu, dass mit keinem der Operationstische 10 und 10' ein gepaarter Betriebszustand hergestellt wird (beziehungsweise der gepaarte Betriebszustand aufgelöst werden würde, wenn ein solcher zuvor vorgelegen hätte). Dementsprechend sendet der Fußschalter 50 in dem Figur 8 gezeigten Zustand, in dem das dem Steuerbefehl "RÜCKEN HOCH" zugeordnete Bedienelement 58 betätigt bleibt, den Steuerbefehl "RÜCKEN HOCH" (S1) nicht aus. Vielmehr wird weiterhin der Anforderungsbefehl X ausgesendet.

### Bezugszeichenliste

- 10, 10': Operationstisch
- 12: Standfläche
- 14: Tischsäule
- 16, 18, 20: Segmente der Patientenlagerfläche
- 22: zweite Infrarot-Sende-Empfangs-Einheit
- 50: Fußschalter
- 52: erste Infrarot-Sende-Empfangs-Einheit
- 54, 56, 58, 60, 62, 64: Bedienelemente
- 66: Bewegungssensor

## Patentansprüche

1. Verfahren zum fernbetätigten Steuern von medizinischen Geräten (10) mittels einer Fernbedienungsvorrichtung (50), bei dem:
zwischen einer in der Fernbedienungsvorrichtung (50) enthaltenen ersten Infrarot-Sende-Empfangs-Einheit (52) und einer in dem jeweiligen medizinischen Gerät (10) enthaltenen zweiten Infrarot-Sende-Empfangs-Einheit (22) ein gepaarter Betriebszustand hergestellt wird, in dem eine von der ersten Infrarot-Sende-Empfangs-Einheit (52) an die zweite Infrarot-Sende-Empfangs-Einheit (22) gesendete Fernbedienungsadresse gleich einer für die zweite Infrarot-Sende-Empfangs-Einheit (22) voreingestellten Geräteadresse ist;
das Vorliegen des gepaarten Betriebszustands geprüft wird; und
bei Vorliegen des gepaarten Betriebszustands das medizinische Gerät (10) in Abhängigkeit eines Steuerbefehls (S1, S2) gesteuert wird, der bei Betätigen der Fernbedienungsvorrichtung (50) von der ersten Infrarot-Sende-Empfangs-Einheit (52) an die zweite Infrarot-Sende-Empfangs-Einheit (22) gesendet wird;
**dadurch gekennzeichnet, dass** der gepaarte Betriebszustand hergestellt wird, indem bei Inbetriebnahme der Fernbedienungsvorrichtung (50) von der ersten Infrarot-Sende-Empfangs-Einheit (52) ein Anforderungsbefehl (X) an die zweite Infrarot-Sende-Empfangs-Einheit (22) gesendet wird, bei Empfang des Anforderungsbefehls (X) von der zweiten Infrarot-Sende-Empfangs-Einheit (22) die voreingestellte Geräteadresse (15) an die erste Infrarot-Sende-Empfangs-Einheit (52) gesendet wird und die von der ersten Infrarot-Sende-Empfangs-Einheit (52) empfangene Geräteadresse als Fernbedienungsadresse übernommen wird und somit die Fernbedienungsadresse gleich der Geräteadresse ist.

2. Verfahren nach Anspruch 1, **dadurch gekennzeichnet, dass** das Vorliegen des gepaarten Betriebszustands geprüft wird, indem die Geräteadresse, nachdem sie von der ersten Infrarot-Sende-Empfangs-Einheit (52) als Fernbedienungsadresse übernommen worden ist, von der zweiten Infrarot-Sende-Empfangs-Einheit (22) zyklisch ausgesendet wird und von der Fernbedienungsvorrichtung (50) geprüft wird, ob die erste Infrarot-Sende-Empfangs-Einheit (52) die Geräteadresse zyklisch empfängt.

3. Verfahren nach Anspruch 1 oder 2, **dadurch gekennzeichnet, dass** das Vorliegen des gepaarten Betriebszustands geprüft wird, indem die Fernbedienungsvorrichtung (50) erfasst, ob die erste Infrarot-Sende-Empfangs-Einheit (52) keine andere als die übernommene Geräteadresse empfängt.

4. Verfahren nach einem der vorhergehenden Ansprüche, **dadurch gekennzeichnet, dass** das Vorliegen des gepaarten Betriebszustands geprüft wird, indem eine Bewegung der Fernbedienungsvorrichtung (50) erfasst wird.

5. Verfahren nach einem der vorhergehenden Ansprüche, **dadurch gekennzeichnet, dass** die von der ersten Infrarot-Sende-Empfangs-Einheit (52) übernommene Geräteadresse in der Fernbedienungsvorrichtung (50) gespeichert wird.

6. Verfahren nach Anspruch 5, **dadurch gekennzeichnet, dass** bei einer nächsten Inbetriebnahme der Fernbedienungsvorrichtung (50) die bei der vorhergehenden Inbetriebnahme gespeicherte Geräteadresse von der ersten Infrarot-Sende-Empfangs-Einheit (52) als Fernbedienungsadresse an die zweite Infrarot-Sende-Empfangs-Einheit (22) gesendet wird.

7. Verfahren nach Anspruch 6, **dadurch gekennzeichnet, dass** für den Fall, dass die bei der nächsten Inbetriebnahme der Fernbedienungsvorrichtung (50) an die zweite Infrarot-Sende-Empfangs-Einheit (22) gesendete Fernbedienungsadresse verschieden von der für die zweite Infrarot-Sende-Empfangs-Einheit (22) voreingestellten Geräteadresse ist, von der Fernbedienungsvorrichtung (50) eine Benutzeraufforderung ausgegeben wird, die Geräteadresse zu betätigen, und bei Bestätigung die Geräteadresse von der ersten Infrarot-Sende-Empfangs-Einheit (52) als Fernbedienungsadresse übernommen wird.

8. Verfahren nach einem der vorhergehenden Ansprüche, **dadurch gekennzeichnet, dass** bei Vorliegen des gepaarten Betriebszustands bei jeder Betätigung der Fernbedienungsvorrichtung (50) der Steuerbefehl (S1, S2) zusammen mit der Fernbedienungsadresse von der ersten Infrarot-Sende-Empfangs-Einheit (52) an die zweite Infrarot-Sende-Empfangs-Einheit (22) gesendet wird.

9. Verfahren nach einem der vorhergehenden Ansprüche, **dadurch gekennzeichnet, dass** als Fernbedienungsvorrichtung ein Fußschalter (50) verwendet wird.

10. Verfahren nach einem der vorhergehenden Ansprüche, **dadurch gekennzeichnet, dass** als medizinische Geräte Operationstische (10) verwendet werden.

11. Einrichtung mit Mitteln eingerichtet zur Durchführung des Verfahrens nach einem der vorhergehenden Ansprüche.

## Claims

1. A method for remote controlling of medical apparatus (10) by means of a remote control device (50), wherein:
between a first infrared transceiver unit (52) included in the remote control device (50) and a second infrared transceiver unit (22) included in the respective medical apparatus (10), a paired operating state is established, where a remote control address sent from the first infrared transceiver unit (52) to the second infrared transceiver unit (22) is equal to a device address preset for the second infrared transceiver unit (22); the presence of the paired operating state is verified; and
in the event of the presence of the paired operating state, the medical apparatus (10) is controlled based on a control command (S1, S2) which is sent from the first infrared transceiver unit (52) to the second infrared transceiver unit (22) when actuating the remote control device (50);
**characterized in that** the paired operating state is established by sending a request command (X) from the first infrared transceiver unit (52) to the second infrared transceiver unit (22) upon start-up of the remote control device (50), by sending the preset device address (15) from the second infrared transceiver unit (22) to the first infrared transceiver unit (52) when receiving the request command (X) and by applying the device address received from the first infrared transceiver unit (52) as a remote control address, the remote control address thus being equal to the device address.

2. The method according to claim 1, **characterized in that** the presence of the paired operating state is verified by cyclically transmitting the device address from the second infrared transceiver unit (22), after it has been applied by the first infrared transceiver unit (52) as remote control address, and by verifying, by the remote control device (50), whether the first infrared transceiver unit (52) cyclically receives the device address.

3. The method according to claim 1 or 2, **characterized in that** the presence of the paired operating state is verified by detecting, by the remote control device (50), whether the first infrared transceiver unit (52) does not receive any other than the device address applied.

4. The method according to one of the preceding claims, **characterized in that** the presence of the paired operating state is verified by detecting a movement of the remote control device (50) .

5. The method according to one of the preceding claims, **characterized in that** the device address applied by the first infrared transceiver unit (52) is saved in the remote control device (50).

6. The method according to claim 5, **characterized in that**, in the event of a subsequent start-up of the remote control device (50), the device address saved during the preceding start-up is sent from the first infrared transceiver unit (52) as remote control address to the second infrared transceiver unit (22).

7. The method according to claim 6, **characterized in that**, in case the remote control address sent during the subsequent start-up of the remote control device (50) to the second infrared transceiver unit (22) is different from the device address preset for the second infrared transceiver unit (22), a user prompt to confirm the device address is output by the remote control device (50) and, upon confirmation, the device address is applied by the first infrared transceiver unit (52) as remote control address.

8. The method according to one of the preceding claims, **characterized in that**, in the event of the presence of the paired operating state, the control command (S1, S2) together with the remote control address is sent from the first infrared transceiver unit (52) to the second infrared transceiver unit (22) each time the remote control device (50) is actuated.

9. The method according to one of the preceding claims, **characterized in that** a foot switch (50) is used as remote control device.

10. The method according to one of the preceding claims, **characterized in that** operating tables (10) are used as medical apparatus.

11. An arrangement with means, equipped for performing the method according to one of the preceding claims.

## Revendications

1. Procédé de commande à distance d'appareils médicaux (10) au moyen d'une télécommande (50), dans lequel :
entre une première unité d'émission-réception infrarouge (52) contenue dans la télécommande (50) et une seconde unité d'émission-réception infrarouge (22) contenue dans l'appareil médical respectif (10), un mode de fonctionnement apparié est établi, dans lequel une adresse de télécommande envoyée par la première unité d'émission-réception infrarouge (52) à la seconde unité d'émission-réception infrarouge (22) est égale à une adresse d'appareil préréglée pour la seconde unité d'émission-réception infrarouge (22) ;
la présence de l'état de fonctionnement apparié est vérifiée ; et
en présence de l'état de fonctionnement apparié, l'appareil médical (10) est commandé en fonction d'une instruction de commande (S1, S2), qui est envoyée lors de l'actionnement de la télécommande (50) de la première unité d'émission-réception infrarouge (52) à la seconde unité d'émission-réception infrarouge (22) ;
**caractérisé en ce que** l'état de fonctionnement apparié est établi par le fait que lors de la mise en service de la télécommande (50), une instruction de demande (X) est envoyée par la première unité d'émission-réception infrarouge (52) à la seconde unité d'émission-réception infrarouge (22), lors de la réception de l'instruction de demande (X) par la seconde unité d'émission-réception infrarouge (22), l'adresse d'appareil préréglée (15) est envoyée à la première unité d'émission-réception infrarouge (52) et l'adresse d'appareil reçue par la première unité d'émission-réception infrarouge (52) est appliquée en tant qu'adresse de télécommande et ainsi l'adresse de télécommande est égale à l'adresse d'appareil.

2. Procédé selon la revendication 1, **caractérisé en ce que** la présence de l'état de fonctionnement apparié est vérifiée, par le fait que l'adresse d'appareil, après avoir été appliquée par la première unité d'émission-réception infrarouge (52) en tant qu'adresse de télécommande, est émise cycliquement par la seconde unité d'émission-réception infrarouge (22) et la télécommande (50) vérifie si la première unité d'émission-réception infrarouge (52) reçoit cycliquement l'adresse d'appareil.

3. Procédé selon la revendication 1 ou 2, **caractérisé en ce que** la présence de l'état de fonctionnement apparié est vérifiée, par le fait que la télécommande (50) détecte si la première unité d'émission-réception infrarouge (52) ne reçoit pas d'autre adresse d'appareil que celle appliquée.

4. Procédé selon l'une des revendications précédentes, **caractérisé en ce que** la présence de l'état de fonctionnement apparié est vérifiée, par le fait qu'un déplacement de la télécommande (50) est détecté.

5. Procédé selon l'une des revendications précédentes, **caractérisé en ce que** l'adresse d'appareil appliquée par la première unité d'émission-réception infrarouge (52) est enregistrée dans la télécommande (50).

6. Procédé selon la revendication 5, **caractérisé en ce que** lors d'une prochaine mise en service de la télécommande (50), l'adresse d'appareil enregistrée lors de la mise en service précédente est envoyée en tant qu'adresse de télécommande de la première unité d'émission-réception infrarouge (52) à la seconde unité d'émission-réception infrarouge (22).

7. Procédé selon la revendication 6, **caractérisé en ce que** pour le cas où l'adresse de télécommande envoyée lors de la prochaine mise en service de la télécommande (50) à la seconde unité d'émission-réception infrarouge (22) est différente de l'adresse d'appareil préréglée pour la seconde unité d'émission-réception infrarouge (22), une demande utilisateur est émise par la télécommande (50), de confirmer l'adresse appareil, et en cas de confirmation l'adresse d'appareil est appliquée par la première unité d'émission-réception infrarouge (52) en tant qu'adresse de télécommande.

8. Procédé selon l'une des revendications précédentes, **caractérisé en ce qu'**en présence de l'état de fonctionnement apparié à chaque actionnement de la télécommande (50) l'instruction de commande (S1, S2) est envoyée conjointement avec l'adresse de télécommande de la première unité d'émission-réception infrarouge (52) à la seconde unité d'émission-réception infrarouge (22).

9. Procédé selon l'une des revendications précédentes, **caractérisé en ce qu'**un commutateur à pédale (50) est utilisé en tant que télécommande.

10. Procédé selon l'une des revendications précédentes, **caractérisé en ce que** des tables d'opération (10) sont utilisées en tant qu'appareils médicaux.

11. Dispositif aménagé avec des moyens pour la réalisation du procédé selon l'une des revendications précédentes.
